# EUROPÄISCHE PATENTANMELDUNG

(11) **EP 4 311 570 A1**
(43) Veröffentlichungstag der Anmeldung: **31.01.2024**
(21) Anmeldenummer: 23187514.7
(22) Anmeldetag: 25.07.2023
(51) Int. Cl.: A61M 39/24, A61M 39/22

(54) **MEDIZINPRODUKT, FUNKTIONSTEIL FÜR EIN MEDIZINPRODUKT UND VERFAHREN ZUM STERILISIEREN UND/ODER ZUM HERSTELLEN VON STERILISATIONSBESTÄNDIGKEIT EINES MEDIZINPRODUKTS ODER FUNKTIONSTEILS**

(30) Priorität: 28.07.2022 DE 102022207782
(71) Anmelder: B. Braun Melsungen AG, 34212 Melsungen (DE)
(72) Erfinder: Arnaldi, Manuel, 3437 Rüderswil (CH); Gläß, Liesa, 6170 Schüpfheim (CH)
(74) Vertreter: Patentanwälte Ruff, Wilhelm, Beier, Dauster & Partner mbB

(57) **Zusammenfassung**

Die Erfindung betrifft ein Medizinprodukt, aufweisend ein elastisch deformierbares Funktionsteil mit einer öffnungsfähigen Schlitzanordnung, wobei sich die Schlitzanordnung bei einer elastischen Deformation des elastisch deformierbaren Funktionsteils aufweitet oder öffnet und bei Wegfall der elastischen Deformation wieder schließt, wobei Schlitzwandungsflächen der Schlitzanordnung mit einer Zusammensetzung beschichtet sind, wobei die Zusammensetzung ein Silikonöl und einen Verdicker aufweist.

Die Erfindung betrifft ferner ein elastisch deformierbares Funktionsteil für ein Medizinprodukt sowie ein Verfahren zum Sterilisieren und/oder zum Herstellen von Sterilisationsbeständigkeit eines Medizinprodukts oder eines elastisch deformierbaren Funktionsteils.

## Beschreibung

### ANWENDUNGSGEBIET UND STAND DER TECHNIK

Die Erfindung betrifft ein Medizinprodukt, das ein elastisch deformierbares Funktionsteil mit einer öffnungsfähigen Schlitzanordnung aufweist, ein entsprechendes Funktionsteil für ein Medizinprodukt sowie ein Verfahren zum Sterilisieren und/oder zum Herstellen von Sterilisationsbeständigkeit eines solchen Medizinprodukts oder Funktionsteils.

Gamma-Bestrahlung stellt ein gängiges Sterilisationsverfahren für Medizinprodukte dar. Weisen die Medizinprodukte ein strahlenvernetzbares Material, wie beispielsweise Silikonelastomere, auf, reagieren diese Materialien bei Einwirkung energiereicher Strahlung mit Vernetzungsreaktionen. Im Falle von Schlitzventilen kann dies zu einem unerwünschten Zuwachsen der Schlitze, auch als Heilen bezeichnet, führen.

Um ein solches Heilen von Schlitzen zu verhindern, werden üblicherweise Additive, zugesetzt. Nachteilig ist, dass eine solche Additivierung in der Regel zu opaken Materialien führt. Für viele Anwendungen, beispielsweise Verbindungseinrichtungen im Bereich der medizinischen Infusionstherapie, ist jedoch der Erhalt von Transparenz einzelner Funktionsteile, wie beispielsweise Ventilkörper, für den bestimmungsgemäßen Gebrauch unbedingt erforderlich. Ferner ist problematisch, dass der Zusatz fester Additive grundlegende Änderungen von Materialeigenschaften bewirken kann.

Neben festen Additiven kommen auch flüssige Additive als Trennmittel zum Einsatz. Diese weisen jedoch keine ausreichende Benetzbarkeit und insbesondere Ortsbeständigkeit auf, um ihre Funktion dauerhaft ausüben zu können. Strahlensterilisation, Alterungsprozesse sowie Transportvorgänge bringen in der Regel erhöhte Temperaturen mit sich, wodurch sich die Trennwirkung flüssiger Trennmittel über die Zeit häufig als zu gering erweist.

### AUFGABE UND LÖSUNG

Der vorliegenden Erfindung liegt daher die Aufgabe zugrunde, ein Medizinprodukt sowie ein Funktionsteil für ein Medizinprodukt bereitzustellen, das im Zusammenhang von gattungsgemäßen Medizinprodukten bzw. Funktionsteilen bekannte Nachteile, insbesondere ein Zuwachsen von Schlitzen und/oder Verlust von Transparenz, teilweise oder vollständig verhindert. Weiter liegt der Erfindung die Aufgabe zugrunde, ein Verfahren zum Sterilisieren und/oder zum Herstellen von Sterilisationsbeständigkeit eines Medizinprodukts oder Funktionsteils für ein Medizinprodukt bereitzustellen, das die eingangs genannten Nachteile teilweise oder vollständig umgeht.

Diese Aufgabe wird gelöst durch ein Medizinprodukt gemäß unabhängigem Anspruch 1, durch ein Funktionsteil gemäß Anspruch 17 sowie durch ein Verfahren gemäß Anspruch 18. Bevorzugte Ausgestaltungen der Erfindung sind in den abhängigen Ansprüchen sowie in der Beschreibung definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Medizinprodukt.

Das Medizinprodukt weist ein elastisch deformierbares Funktionsteil mit einer öffnungsfähigen Schlitzanordnung auf, wobei sich die Schlitzanordnung bei einer elastischen Deformation des elastisch deformierbaren Funktionsteils aufweitet, öffnet oder aufklappt und bei Wegfall der elastischen Deformation wieder schließt. Vorzugsweise weitet sich die Schlitzanordnung bei der elastischen Deformation des elastisch deformierbaren Funktionsteils derart auf oder öffnet sich die Schlitzanordnung bei der elastischen Deformation des elastisch deformierbaren Funktionsteils derart, dass sie einen Durchfluss eines Fluids, insbesondere einer Flüssigkeit, bevorzugt medizinischen Lösung, oder eines Gases, durch das elastisch deformierbare Funktionsteil hindurch freigibt. Eine elastische Deformation des elastisch deformierbaren Funktionsteils kann beispielsweise beim Konnektieren des Medizinprodukts mit einer Infusionsspritze, (Anschluss-)Konnektoren, insbesondere Luer-Slipverbindungen und/oder Luer-Lockverbindungen gemäß ISO80369-7, einem Schlauch oder Schlauchsystem oder Ähnlichem, insbesondere eines medizinischen Infusionssystems, erfolgen.

Das Medizinprodukt zeichnet sich besonders dadurch aus, dass Schlitzwandungsflächen der Schlitzanordnung mit einer Zusammensetzung versehen oder beschichtet, insbesondere benetzt, sind, die ein Öl und einen Verdicker, vorzugsweise ein bioverträgliches Öl und einen bioverträglichen Verdicker, aufweist oder aus einem Öl und einem Verdicker, vorzugsweise einem bioverträglichen Öl und einem bioverträglichen Verdicker, besteht.

Vorzugsweise sind die Schlitzwandungsflächen, insbesondere nur die Schlitzwandungsflächen, der Schlitzanordnung wenigstens teilweise, insbesondere nur teilweise oder vollständig, d.h. vollflächig bzw. durchgehend, mit der Zusammensetzung versehen oder beschichtet.

Die Schlitzwandungsflächen sind in einer Schließstellung der Schlitzanordnung vorzugsweise wenigstens abschnittsweise, insbesondere nur abschnittsweise oder, was bevorzugt ist, durchgehend, insbesondere entlang einer Fluiddurchtrittsachse, insbesondere Mittellängsachse, des elastisch deformierbaren Formteils aneinanderliegend angeordnet oder positioniert. In einer Öffnungsstellung der Schlitzanordnung sind die Schlitzwandungsflächen oder Schlitzwandungen vorzugsweise auseinandergedrückt, beispielsweise durch eine Infusionsspritze, (Anschluss-) Konnektoren, insbesondere Luer-Slipverbindungen und/oder Luer-Lockverbindungen gemäß ISO80369-7, einen Schlauch oder ein Schlauchsystem oder Ähnliches, insbesondere eines medizinischen Infusionssystems.

Unter dem Ausdruck "Funktionsteil" soll im Sinne der vorliegenden Erfindung eine Komponente oder ein Bauteil oder ein Bauteilelement des Medizinprodukts verstanden werden.

Unter dem Ausdruck "Schlitzwandungsflächen" sollen im Sinne der vorliegenden Erfindung Flächen oder Oberflächen von Wandungen, die einen Schlitz bilden oder definieren oder an der Ausbildung eines Schlitzes beteiligt sind, verstanden werden.

Unter dem Ausdruck "Trennmittel" soll im Sinne der vorliegenden Erfindung eine Substanz oder eine Zusammensetzung verstanden werden, die in der Lage ist, ein Zuwachsen, insbesondere strahleninduziertes Zuwachsen, von Schlitzen zu reduzieren oder (vollständig) zu vermeiden.

Unter dem Ausdruck "bioverträgliches Öl" oder "bioverträglicher Verdicker" " soll im Sinne der vorliegenden Erfindung ein Öl oder ein Verdicker, verstanden werden, das oder der auf dem Gebiet der Medizin, insbesondere auf medizinischen Gebieten mit direktem oder indirektem Patientenkontakt, ohne gesundheitliche Bedenken oder Risiken verwendet werden kann.

Unter dem Ausdruck "Verdicker" soll im Sinne der vorliegenden Erfindung eine Substanz verstanden werden, welche die Konsistenz oder Viskosität der Zusammensetzung erhöht.

Die Erfindung beruht auf dem überraschenden Befund, dass sich die erfindungsgemäß vorgesehene Zusammensetzung als Trennmittel zur Verhinderung von Heilungsphänomenen, d.h. eines Zuwachsens von Schlitzen, bei elastisch deformierbaren Funktionsteilen sowie Medizinprodukten, die derartige Funktionsteile aufweisen, eignen. Aufgrund ihrer Bestandteile zeichnet sich die erfindungsgemäße Zusammensetzung vorteilhafterweise durch eine ausreichende Benetzungsfähigkeit und insbesondere Ortsbeständigkeit aus. Dadurch kann sie wiederum ihre Trennfunktion langfristig oder dauerhaft entfalten. Bei Einwirkung von Strahlung, insbesondere Gamma-Strahlung, Beta-Strahlung, Röntgen-Strahlung oder Elektronenstrahl-Strahlung (e-beam-Strahlung), ist somit ein besserer Schutz vor strahlungsinduzierten Vernetzungsphänomenen erzielbar. Insbesondere von Vorteil ist dabei, dass die Erfindung die Durchführung einer Strahlensterilisation, insbesondere Gammastrahlensterilisation, mit einer Strahlendosis von wenigstens 18 kGy, insbesondere wenigstens 32 kGy, insbesondere wenigstens 40 kGy, ohne (nennenswertes) Zuwachsen von Schlitzen ermöglicht. Von Vorteil ist ferner, dass durch die Erfindung eine Erhöhung der Alterungsresistenz erzielbar ist.

In Ausgestaltung der Erfindung ist das Öl ausgewählt aus der Gruppe bestehend aus Silikonöl, alkyliertem Naphthalin (AN), Chlortrifluoroethylen (CTFE), Esteröl, Mineralöl, insbesondere synthetisches Mineralöl, multialkyliertes Cyclopentan (MAC), Polyalphaolefin (PAO), Polyphenylether (PPE), Polyglykolöl (PG), perfluoriertes Polyetheröl (PFPE), Triglyceride und Mischungen von wenigstens zwei der vorgenannten Öle.

Die vorgenannten Öle haben insbesondere den Vorteil, dass sie besonders reaktionsarm gegenüber Strahlensterilisation sind, wodurch die Trennwirkung der erfindungsgemäßen Zusammensetzung zusätzlich optimiert werden kann.

In weiterer Ausgestaltung der Erfindung ist das Öl ein Silikonöl. Insbesondere kann es sich bei dem Silikonöl um ein fluoriertes Silikonöl, insbesondere teil- oder perfluoriertes Silikonöl, handeln.

Besonders bevorzugt ist das Öl ein perfluoriertes Silikonöl. Insoweit hat sich vorteilhafterweise herausgestellt, dass ein Silikonöl mit höherem Fluoranteil weniger auf Strahlung, insbesondere Gamma-Strahlung, Beta-Strahlung, Röntgen-Strahlung oder Elektronenstrahl-Strahlung (e-beam-Strahlung), reagiert und somit ein besserer Schutz vor strahlungsinduzierten Vernetzungsphänomenen erzielbar ist.

In weiterer Ausgestaltung der Erfindung weist das Öl einen Anteil von 0.1 Gew.-% bis 99.9 Gew.%, insbesondere 10 Gew.-% bis 90 Gew.-%, vorzugsweise 20 Gew.-% bis 80 Gew.-%, auf, bezogen auf das Gesamtgewicht der Zusammensetzung.

Bei dem Verdicker handelt es sich vorzugsweise um einen Feststoff.

In weiterer Ausgestaltung der Erfindung ist der Verdicker eine Metallseife, insbesondere Leichtmetallseife, besonders bevorzugt Alkalimetallseife und/oder eine Erdalkalimetallseife.

Unter dem Ausdruck "Leichtmetall" soll im Sinne der vorliegenden Erfindung Aluminium, Alkalimetalle, insbesondere Lithium, Natrium, Kalium, Rubidium, Cäsium oder Francium, Erdalkalimetalle, insbesondere Beryllium, Magnesium, Calcium, Strontium oder Barium, oder Mischungen aus wenigstens zwei der vorgenannten Metalle verstanden werden.

Unter dem Ausdruck "Metallseife" soll im Sinne der vorliegenden Erfindung ein Metallsalz einer Fettsäure, eine sogenannte Einfachseife, oder ein Metallsalz eines Fettsäuregemisches, eine sogenannte Komplexseife, verstanden werden. Die Fettsäure kann insbesondere ausgewählt sein aus der Gruppe bestehend aus Ameisensäure, Essigsäure, Propionsäure, Buttersäure, Valeriansäure, Capronsäure, Önanthsäure, Caprylsäure, Pelargonsäure, Caprinsäure, Laurinsäure, Myristinsäure, Palmitinsäure, Margarinsäure, Stearinsäure, Arachinsäure, Behensäure, Lignocerinsäure, Cerotinsäure, Montansäure, Melissinsäure, Laccersäure, Geddinsäure, Undecylensäure, Myristoleinsäure, Palmitoleinsäure, Margaroleinsäure, Petroselinsäure, Ölsäure (OA), Elaidinsäure, Vaccensäure, Gadoleinsäure, Gondosäure, Cetoleinsäure, Erucasäure, Nervonsäure, Linolsäure (LA), Alpha-Linolensäure, Gamma-Linolensäure, Calendulasäure, Punicinsäure, Alpha-Eleostearinsäure, Beta-Eleostearinsäure, Stearidonsäure, Arachidonsäure, Eicosapentaensäure, Docosadiensäure, Docosatetraensäure, Docosapentaensäure, Docosahexaensäure, Tetracosahexaensäure und Mischungen von wenigstens zwei der vorgenannten Fettsäuren.

Insbesondere kann die Metallseife ausgewählt sein aus der Gruppe bestehend aus Einfachseifen, insbesondere Aluminiumseife, Bariumseife, Calciumseife, Lithiumseife oder Natriumseife, und Komplexseifen, insbesondere Aluminiumkomplexseife, Bariumkomplexseife, Calciumkomplexseife, Lithiumkomplexseife oder Natriumkomplexseife, und Mischungen von wenigstens zwei der vorgenannten Metallseifen.

In weiterer Ausgestaltung der Erfindung ist der Verdicker ausgewählt aus der Gruppe bestehend aus Polytetrafluorethylen (PTFE), anorganischer Verdicker, insbesondere Bentonit, Polyharnstoff, Silika, Glucose, Ascorbinsäure, Stärke und Mischungen von wenigstens zwei der vorgenannten Verdicker.

In weiterer Ausgestaltung der Erfindung ist der Verdicker Polytetrafluorethylen (PTFE) oder Silika oder eine Mischung aufweisend oder bestehend aus Polytetrafluorethylen (PTFE) und Silika.

In weiterer Ausgestaltung der Erfindung weist der Verdicker einen Anteil von 0.1 Gew.-% bis 99.9 Gew.-%, insbesondere 10 Gew.-% bis 90 Gew.-%, vorzugsweise 20 Gew.-% bis 80 Gew.-%, auf, bezogen auf das Gesamtgewicht der Zusammensetzung.

Die vorgenannten Verdicker haben sich als besonders vorteilhaft herausgestellt, um der erfindungsgemäßen Zusammensetzung eine gewisse Benetzungsfähigkeit und insbesondere Ortsbeständigkeit zu verleihen, wodurch sich die Trennwirkung der Zusammensetzung besonders gut entfalten kann.

In weiterer Ausgestaltung der Erfindung weist die Zusammensetzung ferner einen Feststoff, insbesondere in Form eines Pulvers, auf, der kein Verdicker ist. Der Feststoff ist vorzugsweise ausgewählt aus der Gruppe bestehend aus Salzen, Sacchariden, Vitaminen und Mischungen von wenigstens zwei der vorgenannten Feststoffe.

In weiterer Ausgestaltung der Erfindung weist der Feststoff einen Anteil von 0.01 Gew.-% bis 99.99 Gew.-%, insbesondere 1 Gew.-% bis 99 Gew.-%, vorzugsweise 10 Gew.-% bis 90 Gew.%, auf, bezogen auf das Gesamtgewicht der Zusammensetzung.

Der Feststoff kann eine Korngröße oder einen mittleren Partikeldurchmesser von < (gesprochen: kleiner) 400 µm, insbesondere 1 µm bis 50 µm, vorzugsweise 0,01 µm bis 10 µm, aufweisen.

Bei den Salzen kann es sich insbesondere um ein Alkalimetallsalz, vorzugsweise ausgewählt aus der Gruppe bestehend aus Alkalimetallchlorid, Alkalimetallbromid, Alkalimetalliodid und Mischungen von wenigstens zwei der vorgenannten Alkalimetallsalze, handeln.

Das Alkalimetallchlorid kann insbesondere ausgewählt sein aus der Gruppe bestehend aus Lithiumchlorid, Natriumchlorid, Kaliumchlorid und Mischungen von wenigstens zwei der vorgenannten Alkalimetallchloride.

Das Alkalimetallbromid kann insbesondere ausgewählt sein aus der Gruppe bestehend aus Lithiumbromid, Natriumbromid, Kaliumbromid und Mischungen von wenigstens zwei der vorgenannten Alkalimetallbromide.

Das Alkalimetalliodid kann insbesondere ausgewählt sein aus der Gruppe bestehend aus Lithiumiodid, Natriumiodid, Kaliumiodid und Mischungen von wenigstens zwei der vorgenannten Alkalimetalliodide.

Besonders bevorzugt handelt es sich bei dem Feststoff um Natriumchlorid.

Bei Verwendung eines oder mehrerer der vorgenannten Salze, insbesondere von Natriumchlorid, lassen sich die erfindungsgemäßen Vorteile zusätzlich verbessern.

Bei den Sacchariden kann es sich grundsätzlich um Monosaccharide, Oligosaccharide, wie beispielsweise Disaccharide, oder Polysaccharide, oder um eine Mischung von wenigstens zwei der vorgenannten Saccharide handeln.

Bevorzugt handelt es sich bei den Sacchariden um Glucose.

Durch die Verwendung von Sacchariden, insbesondere Glucose, lassen sich die erfindungsgemäßen Vorteile (ebenfalls) zusätzlich optimieren.

Bei den Vitaminen kann es sich insbesondere um Ascorbinsäure, vorzugsweise L-(+)-Ascorbinsäure, d.h. Vitamin C, handeln. Die erfindungsgemäßen Vorteile lassen sich (auch) bei Verwendung von Ascorbinsäure, vorzugsweise L-(+)-Ascorbinsäure bzw. Vitamin C, zusätzlich verbessern.

In weiterer Ausgestaltung der Erfindung ist die Zusammensetzung eine zähflüssige oder pastöse Zusammensetzung. Durch eine solche Zusammensetzung kommen die erfindungsgemäßen Vorteile besonders gut zur Geltung. Insbesondere können hierdurch die einleitend im Zusammenhang von flüssigen Additiven beschriebenen Nachteile besonders gut vermieden werden. Vorzugsweise liegt die Zusammensetzung in Form einer Schmierfettzusammensetzung vor.

Weiter bevorzugt weist die Zusammensetzung oder das Öl der Zusammensetzung eine Viskosität von 100 mm²/s bis 1000000 mm²/s, insbesondere 200 mm²/s bis 12500 mm²/s, vorzugsweise 300 mm²/s bis 2000 mm²/s , auf. Die Viskosität wird vorzugsweise bei 20 °C oder 40 °C bestimmt.

In weiterer Ausgestaltung der Erfindung ist das Funktionsteil wenigstens abschnittsweise, insbesondere nur abschnittsweise oder vollständig, transparent, d.h. lichtdurchlässig, gestaltet. Unter dem Ausdruck "transparent" bzw. "lichtdurchlässig" soll im Sinne der vorliegenden Erfindung die Fähigkeit von Materie, elektromagnetische Wellen mit einer Wellenlänge von 380 nm bis 780 nm hindurchzulassen (Transmission), verstanden werden. Insoweit beruht die Erfindung auf der weiteren überraschenden Erkenntnis, dass die erfindungsgemäß vorgesehene Zusammensetzung im Falle eines transparenten Funktionsteils nicht zu einer opaken Veränderung des Funktionsteils führt. Vielmehr bleibt die Transparenz des elastisch deformierbaren Funktionsteils vorteilhafterweise erhalten.

In weiterer Ausgestaltung der Erfindung weist das elastisch deformierbare Funktionsteil ein elastisch deformierbares Material, insbesondere ein transparentes, d.h. lichtdurchlässiges, elastisch deformierbares Material, auf oder ist das elastisch deformierbare Funktionsteil, insbesondere einteilig, aus einem elastisch deformierbaren Material, insbesondere transparenten, d.h. lichtdurchlässigen, elastisch deformierbaren Material, gefertigt.

Bei dem elastisch deformierbaren Material handelt es sich vorzugsweise um ein Elastomer und/oder thermoplastisches Elastomer.

Das Elastomer kann insbesondere ausgewählt sein aus der Gruppe bestehend aus Silikonelastomer wie Silikonkautschuk, Acrylnitril-Butadien-Kautschuk, hydrierter Acrylnitril-Butadien-Kautschuk, Styrolbutadienkautschuk und Mischungen von wenigstens zwei der vorgenannten Elastomere. Alternativ oder in Kombination kann es sich bei dem Elastomer um ein anderes Polymer handeln, das vernetzungsfähig ist oder in dem Vernetzungsreaktionen überwiegen.

In weiterer Ausgestaltung der Erfindung sind die Schlitzwandungsflächen, insbesondere nur die Schlitzwandungsflächen, der Schlitzanordnung wenigstens teilweise, insbesondere nur teilweise oder vollständig, d.h. durchgehend oder vollflächig, mit der Zusammensetzung beschichtet, insbesondere benetzt. Vorzugsweise ist das elastisch deformierbare Funktionsteil abgesehen von den Schlitzwandungsflächen der Schlitzanordnung frei von der Zusammensetzung. Insbesondere liegt die Zusammensetzung nicht innerhalb des elastisch deformierbaren Funktionsteils, insbesondere dispergiert oder verteilt, vor. Stattdessen befindet sich die Zusammensetzung vorzugsweise ausschließlich auf den Schlitzwandungsflächen der Schlitzanordnung. Dadurch kann vorteilhafterweise das Risiko, dass sich Materialeigenschaften, wie insbesondere Transparenz und/oder mechanische Eigenschaften, des Funktionsteils verändern, zusätzlich reduziert werden.

Weiter können die Schlitzwandungsflächen, insbesondere nur die Schlitzwandungsflächen, der Schlitzanordnung wenigstens teilweise, insbesondere nur teilweise oder vollständig, d.h. durchgehend oder vollflächig, mit 0,01 mg bis 2 mg, insbesondere 0,8 mg bis 1,2 mg, vorzugsweise 1 mg, der Zusammensetzung versehen oder beschichtet sein.

Weiter können die Schlitzwandungsflächen, insbesondere nur die Schlitzwandungsflächen, der Schlitzanordnung wenigstens teilweise, insbesondere nur teilweise oder vollständig, d.h. durchgehend oder vollflächig, in einer Dicke von 0,01 µm bis 800 µm, insbesondere 0,1 µm bis 400 µm, vorzugsweise 1 µm bis 20 µm, mit der Zusammensetzung versehen oder beschichtet sein.

In weiterer Ausgestaltung der Erfindung ist die Schlitzanordnung als Längsschlitzanordnung, d.h. lineare Schlitzanordnung, gestaltet.

Alternativ kann die Schlitzanordnung hiervon abweichend, beispielsweise Y- oder kreuzförmig, d.h. als sogenannte Kreuzschlitzanordnung, gestaltet sein.

In weiterer Ausgestaltung der Erfindung ist das elastisch deformierbare Funktionsteil als Ventilkörper gestaltet.

In weiterer Ausgestaltung der Erfindung weist das elastisch deformierbare Funktionsteil, insbesondere der Ventilkörper, einen Kopfbereich, insbesondere einen deckelförmigen Kopfbereich, auf. Die Schlitzanordnung ist bevorzugt entlang einer Quererstreckung, d.h. quer zu einer Längserstreckung, des Kopfbereiches ausgebildet. Besonders bevorzugt erstreckt sich die Schlitzanordnung, insbesondere mittig, längs einer Längsachse oder Fluiddurchtrittsachse, insbesondere Mittellängsachse, durch den Kopfbereich hindurch. Vorzugsweise schließt sich an den Kopfbereich, insbesondere unmittelbar, ein Mantel des elastisch deformierbaren Funktionsteils an. Ist das elastisch deformierbare Funktionsteil als Ventilkörper gestaltet, was erfindungsgemäß bevorzugt ist, schließt sich an den Kopfbereich, insbesondere unmittelbar, ein Ventilmantel an. Der Ventilmantel weist vorzugsweise einen radialen Querschnitt auf, der bevorzugt eine größere Längserstreckung als Quererstreckung besitzt. Der radiale Querschnitt des Ventilmantels ist bezogen auf eine Längsachse oder Fluiddurchtrittsachse des Ventilkörpers, die vorzugsweise einer Mittellängsachse des Ventilkörpers entspricht. Der Kopfbereich und/oder Mantel, beispielsweise nur der Kopfbereich oder nur der Mantel, des elastisch deformierbaren Funktionsteils, insbesondere Ventilkörpers, können transparent gestaltet sein, d.h. ein transparentes, elastisch deformierbares Material, vorzugsweise ein Elastomer, insbesondere Silikon-Elastomer, und/oder thermoplastisches Elastomer, aufweisen oder aus einem solchen Material gefertigt sein. Bezüglich weiterer geeigneter Materialien sei auf die vorangegangene Beschreibung verwiesen.

Vorzugsweise wird das elastisch deformierbare Funktionsteil für eine Verbindungseinrichtung eines medizinischen Infusionssystems verwendet. Eine entsprechende Verbindungseinrichtung ist beispielsweise aus der EP 3 216 486 A1 bekannt. Die dort in Bezug auf die Verbindungseinrichtung beschriebenen Merkmale und Vorteile werden durch ausdrückliche Bezugnahme zum Inhalt der vorliegenden Beschreibung gemacht.

In weiterer Ausgestaltung der Erfindung handelt es sich bei dem Medizinprodukt um ein Medizinprodukt für die Infusionstherapie. Insbesondere bevorzugt handelt es sich bei dem Medizinprodukt um eine Verbindungseinrichtung, insbesondere eines medizinischen Infusionssystems oder für ein medizinisches Infusionssystem. Das Medizinprodukt, insbesondere die Verbindungseinrichtung, kann beispielsweise als Einwegehahn, Zweiwegehahn, Dreiwegehahn, Vierwegehahn oder als Hahnbank, d.h. als eine Einheit oder ein System mit oder aus hintereinander, d.h. in Reihe angeordneter oder geschalteter, Einwegehähne, gestaltet sein. Vorzugsweise ist das Medizinprodukt, insbesondere die Verbindungseinrichtung, als Zweiwegehahn, Dreiwegehahn, Vierwegehahn oder als Hahnbank gestaltet.

Bevorzugt liegt das Medizinprodukt in sterilisierter, insbesondere strahlensterilisierter, vorzugsweise gammastrahlensterilisierter, betastrahlensterilisierter, röntgenstrahlensterilisierter oder elektronenstrahlensterilisierter Form vor. Vorzugsweise ist das Medizinprodukt durch Bestrahlung, vorzugsweise Gamma-Bestrahlung, Beta-Bestrahlung, Röntgen- Bestrahlung oder Elektronenstrahl-Bestrahlung (e-beam-Bestrahlung), insbesondere mit einer Strahlendosis von 18 kGy bis 100 kGy, bevorzugt 18 kGy bis 40 kGy, insbesondere 18 kGy bis 32 kGy, sterilisiert. Insbesondere bevorzugt kann das Medizinprodukt mit einer Strahlendosis von 32 kGy, vorzugsweise 40 kGy, bis 100 kGy, sterilisiert sein.

Alternativ kann das Medizinprodukt in nicht sterilisierter Form vorliegen.

Gemäß einem zweiten Aspekt betrifft die Erfindung ein elastisch deformierbares Funktionsteil für ein Medizinprodukt.

Das elastisch deformierbare Funktionsteil weist eine öffnungsfähige Schlitzanordnung auf, die sich bei einer elastischen Deformation des elastisch deformierbaren Funktionsteils aufweitet, öffnet oder aufklappt und bei Wegfall der elastischen Deformation wieder schließt. Schlitzwandungsflächen der Schlitzanordnung sind mit einer Zusammensetzung versehen oder beschichtet, insbesondere benetzt, die ein Öl, vorzugsweise bioverträgliches Öl, und einen Verdicker, vorzugsweise bioverträglichen Verdicker, aufweist oder aus einem Öl, vorzugsweise bioverträglichen Öl, und einem Verdicker, vorzugsweise bioverträglichen Verdicker, besteht.

Bezüglich weiterer Merkmale und Vorteile des elastisch deformierbaren Funktionsteils, insbesondere der Schlitzanordnung sowie der Zusammensetzung, sowie des Medizinprodukts wird vollständig auf die entsprechenden, unter dem ersten Erfindungsaspekt gemachten Ausführungen Bezug genommen. Die dort insoweit beschriebenen Merkmale und Vorteile gelten sinngemäß auch für das elastisch deformierbare Funktionsteil gemäß zweitem Erfindungsaspekt.

Gemäß einem dritten Aspekt betrifft die Erfindung ein Verfahren zum Sterilisieren und/oder zum Herstellen von Sterilisationsbeständigkeit eines Medizinprodukts gemäß erstem Erfindungsaspekt oder eines elastisch deformierbaren Funktionsteils gemäß zweitem Erfindungsaspekt. Das Verfahren weist, vorzugsweise in zeitlicher Reihenfolge, nachfolgende Schritte auf:
a) Beschichten oder Beaufschlagen, insbesondere Benetzen, der Schlitzwandungsflächen der Schlitzanordnung mit der Zusammensetzung, die ein Öl und einen Verdicker, vorzugsweise ein bioverträgliches Öl und einen bioverträglichen Verdicker, aufweist oder aus einem Öl, vorzugsweise bioverträglichen Öl, und einem Verdicker, vorzugsweise bioverträglichen Verdicker, besteht, und
b) Sterilisieren des Medizinprodukts oder elastisch deformierbaren Funktionsteils, insbesondere durch Einwirkung von Strahlung, vorzugsweise Gamma-Strahlung, Röntgen-Strahlung, Beta-Strahlung oder Elektronenstrahl-Strahlung (e-beam-Strahlung).

Das Verfahren gemäß drittem Erfindungsaspekt zeichnet sich vorzugsweise dadurch aus, dass Schritt b) zeitlich nach dem Schritt a) durchgeführt wird.

Die Schlitzwandungsflächen der Schlitzanordnung können nur teilweise oder vollständig, d.h. durchgehend bzw. vollflächig, mit der Zusammensetzung beschichtet oder beaufschlagt, insbesondere benetzt, werden.

In weiterer Ausgestaltung der Erfindung werden die Schlitzwandungsflächen, insbesondere nur die Schlitzwandungsflächen, der Schlitzanordnung wenigstens teilweise, insbesondere nur teilweise oder vollständig, d.h. durchgehend oder vollflächig, mit 0,01 mg bis 2 mg, insbesondere 0,8 mg bis 1,2 mg, vorzugsweise 1 mg, der Zusammensetzung versehen oder beschichtet, insbesondere benetzt.

Weiter kann der Schritt b) mit einer Strahlendosis von 18 kGy bis 100 kGy, bevorzugt 18 kGy bis 40 kGy, insbesondere 18 kGy bis 32 kGy, durchgeführt werden. Insbesondere kann der Schritt b) mit einer Strahlendosis von 32 kGy, vorzugsweise 40 kGy, bis 100 kGy durchgeführt werden.

Bezüglich weiterer Merkmale und Vorteile des Verfahrens wird vollständig auf die entsprechenden, unter dem ersten sowie zweiten Erfindungsaspekt gemachten Ausführungen Bezug genommen. Die dort insbesondere in Bezug auf das elastisch deformierbare Funktionsteil sowie das Medizinprodukt beschriebenen Merkmale und Vorteile gelten sinngemäß auch für das Verfahren gemäß drittem Erfindungsaspekt.

Weitere Merkmale und Vorteile der Erfindung ergeben sich aus der nachfolgenden Beschreibung bevorzugter Ausführungsformen anhand von Figuren, Figurenbeschreibungen sowie von Unteransprüchen. Dabei können einzelne Merkmale der Erfindung für sich alleine oder zu mehreren in Kombination miteinander verwirklicht sein. Die bevorzugten Ausführungsformen dienen lediglich der Erläuterung der Erfindung, die in keiner Weise darauf beschränkt sein soll.

### FIGURENKURZBESCHREIBUNGEN

- Fig. 1: zeigt in einer Draufsicht eine Ausführungsform eines erfindungsgemäßen Medizinprodukts in Form einer Verbindungseinrichtung,
- Fig. 2: einen Schnitt durch das Medizinprodukt nach Fig. 1 entlang der Schnittlinie II-II in Fig. 1,
- Fig. 3: eine Ansicht von unten eines als Ventilkörper gestalteten Funktionsteils des Medizinprodukts gemäß Fig. 2,
- Fig. 4: einen Längsschnitt durch den Ventilkörper nach Fig. 3 entlang der Schnittlinie IV-IV in Fig. 3,
- Fig. 5: einen weiteren Längsschnitt durch die Ventilkörper längs der Schnittlinie V-V in Fig. 3,
- Fig. 6: eine Draufsicht auf den Ventilkörper nach den Fig. 3 bis 5,
- Fig. 7 bis 9: in Schnittdarstellungen verschiedene Schritte bei einer Konnektierung einer Komponente an das Medizinprodukt nach Fig. 2 sowie
- Fig. 10a, b: zeigen eine Ausführungsform eines erfindungsgemäßen Verfahrens.

### AUSFÜHRLICHE FIGURENBESCHREIBUNGEN

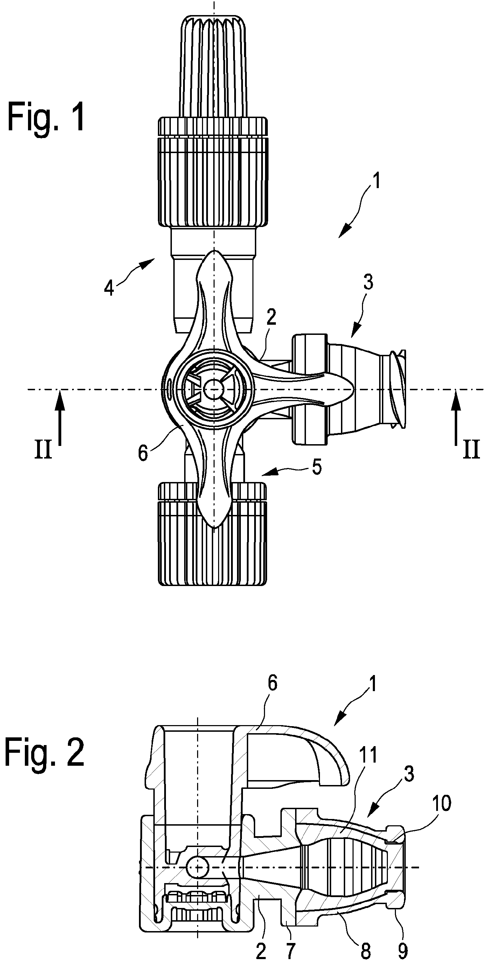

Fig. 1 zeigt schematisch eine Ausführungsform eines erfindungsgemäßen Medizinprodukts in Form einer Verbindungseinrichtung 1 eines medizinischen Infusionssystems. Die Verbindungseinrichtung 1 kann beispielsweise, wie in Fig. 1 gezeigt, als Dreiwegehahn gestaltet sein. Es versteht sich jedoch, dass die Verbindungseinrichtung 1 hiervon abweichend, insbesondere als Einwege- oder Zweiwegehahn oder als Hahnbank, d.h. als eine Einheit oder ein System mit oder aus in Reihe, d.h. hintereinander, geschalteter Einwegehähne, gestaltet sein kann.

Die Verbindungseinrichtung 1 weist ein Gehäuse 2, einen Anschlussstutzen 3 sowie zwei Anschlussbereiche 4, 5 auf. In dem Gehäuse 2 ist ein Stellglied 6 drehbeweglich gelagert. In dem Gehäuse 2 sind ferner insgesamt drei Verbindungskanäle vorgesehen, die je nach Stellung des Stellglieds 6 abgesperrt oder miteinander verbunden werden. Ein Verbindungskanal des Gehäuses 2 führt zu dem Anschlussstutzen 3. Ein weiterer, rechtwinklig angeordneter Verbindungskanal führt zu dem Anschlussbereich 4 und gegenüberliegend hierzu ein dritter Verbindungskanal zu dem Anschlussbereich 5. Einer der beiden Anschlussbereiche 4, 5 ist für den Anschluss einer Patientenleitung vorgesehen. Der andere Anschlussbereich 4, 5 dient dazu, eine Verbindungsleitung zu einem Fluidbehältnis anzuschließen.

Der Anschlussstutzen 3 ist vorzugsweise zum zeitweisen Konnektieren einer Komponente des medizinischen Infusionssystems, wie insbesondere einer Spritze, vorgesehen, um der Patientenleitung beispielsweise (zusätzliche) Medikamente oder Ähnliches zuzuführen. Zusätzlich oder alternativ kann der Anschlussstutzen 3 für eine Konnektion, insbesondere dauerhafte Konnektion (Dauerkonnektion), einer zusätzlichen Leitung vorgesehen sein.

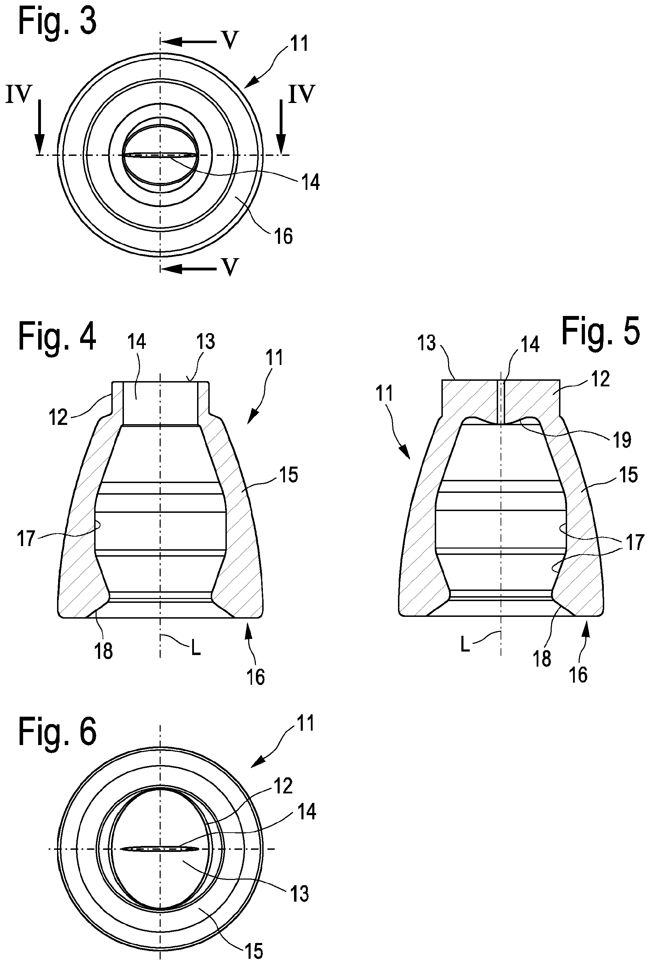

Der Anschlussstutzen 3 wird anhand der nachfolgenden Fig. 3 bis 5 näher erläutert.

Der Anschlussstutzen 3 kann eine formstabile Kappe 8 aufweisen, die an ihrer dem Gehäuse 2 zugewandten Stirnseite mit einem formstabilen Bodenabschnitt 7 des Gehäuses 2 verbunden ist, vorzugsweise fest. Die Kappe 8 ist hülsenförmig gestaltet und weist auf der dem Bodenabschnitt 7 zugewandten Seite einen verdickten Randbereich auf, der mit dem Bodenabschnitt 7, beispielsweise durch Verschweißung, fest verbunden ist. Der Bodenabschnitt 7 ist tellerförmig ausgeführt und ragt relativ zu einer Mittellängsachse L des Anschlussstutzens 3 radial nach außen ab. Der Bodenabschnitt 7 umgibt einen sich zu einem Inneren des Gehäuses 2 konisch verjüngenden oder nicht konisch verjüngenden Kanalabschnitt. Alternativ kann der Kanalabschnitt zylindrisch oder nicht zylindrisch gestaltet sein.

Die Kappe 8 ist an ihrem zu dem Bodenabschnitt 7 abliegenden Stirnbereich mit einem Durchtritt 10 versehen, der durch einen nachfolgend näher beschriebenen Ventilkörper 11 der Verbindungseinrichtung 1 verschließbar ist. Der Durchtritt 10 wird von einem verdickten Randbereich umschlossen, der mit Verbindungsprofilierungen 9 in Form von Luer-Lock-Profilierungen versehen ist.

Der Ventilkörper 11 ist vorzugsweise becher- oder glockenartig gestaltet und insbesondere einstückig aus einem elastisch deformierbaren Material gefertigt. Bei dem elastisch deformierbaren Material handelt es sich vorzugsweise um ein Elastomer oder thermoplastisches Elastomer. Besonders bevorzugt handelt es sich bei dem elastisch deformierbaren Material um ein Silikonelastomer, wie beispielsweise Silikon-Kautschuk. Vorzugsweise ist das elastisch deformierbare Material zudem transparent, d.h. lichtdurchlässig.

Der Ventilkörper 11 weist bevorzugt eine Außenkontur auf, die in einem unbelasteten, d.h. nicht deformierten, Ausgangszustand über eine gesamte Höhe der Kappe 8 bündig und flächig an der Innenkontur der Kappe 8 anliegt. Der Ventilkörper 11 weist einen Kopfbereich, insbesondere deckelförmigen Kopfbereich, 12 auf. Der Kopfbereich 12 kann eine rotationssymmetrische, insbesondere kreisförmige, oder, wie dargestellt, eine rotationsunsymmetrische, insbesondere ovale, Grundfläche aufweisen (siehe insbesondere Figuren 3 und 6). Der Ventilkörper 11 weist ferner einen Ventilmantel 15 auf. Der Ventilmantel 15 schließt an den Kopfbereich 12 an, vorzugsweise unmittelbar. Vorzugsweise ist der Ventilmantel 15 an einem unteren Stirnendbereich mit einem Bodenring 16 versehen. Der Kopfbereich 12 ist mit einer Schlitzanordnung 14 versehen. Eine Oberfläche 13 des Kopfbereichs 12 ist vorzugsweise glatt und eben gestaltet.

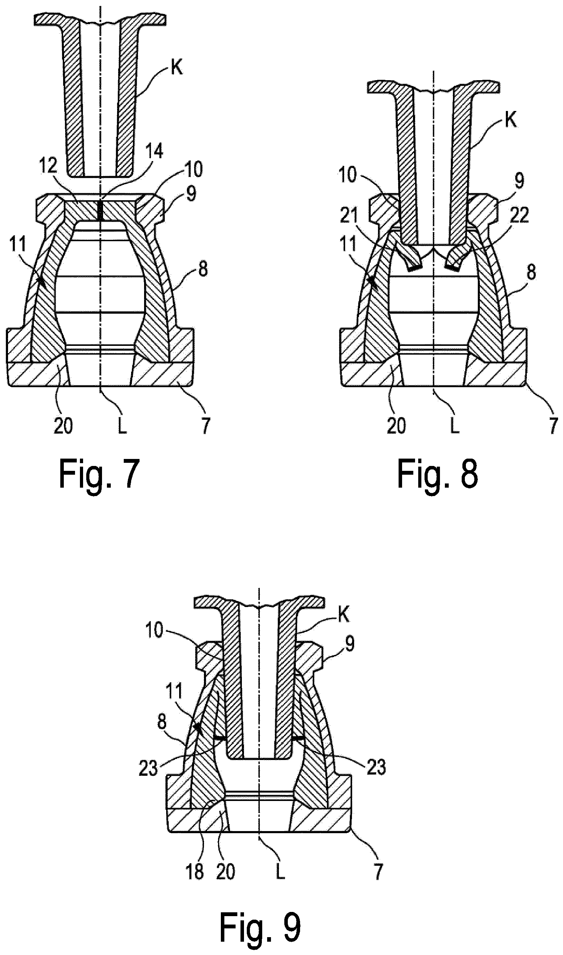

Anhand der Fig. 7 ist erkennbar, dass die Oberfläche 13 des Kopfbereichs 12 in einem unbelasteten Ausgangszustand des Ventilkörpers 11 in der Kappe 8 mit einer Randkante des Durchtritts 10 bündig abschließen kann. Von dieser Randkante des Durchtritts 10 aus erstreckt sich eine schräge Anfasung nach außen bis zu einer Stirnfläche des den Durchtritt 10 definierenden Randbereichs der Kappe 8. In dem unbelasteten Ausgangszustand des Ventilkörpers 11 kann demzufolge die Stirnfläche des Randbereichs der Kappe 8 einschließlich der Oberfläche 13 des Kopfbereichs 12 des Ventilkörpers 11 in einfacher Weise, beispielsweise mittels eines Desinfektionstuchs oder Ähnlichem, durch medizinisches Personal gereinigt und desinfiziert werden.

Der Ventilmantel 15 des Ventilkörpers 11 ist relativ zu der Mittellängsachse L vorzugsweise rotationssymmetrisch ausgeführt. Bevorzugt weist der Ventilmantel 15 eine Wandung auf, die sich ausgehend von dem Kopfbereich 12 bis zu dem Bodenbereich 16 hin verdickt. Die Verdickung kann dabei unstetig und nicht linear erfolgen, wie anhand der beiden dargestellten sichtbaren Kanten erkennbar ist. Die Kanten sind vorzugsweise ringförmig umlaufend. Dabei kann der Ventilmantel 15 einen an den Kopfbereich 12 anschließenden, sich kegelstumpfartig erweiternden ersten Wandungsabschnitt mit gleichbleibender Dicke aufweisen. An diesen ersten Wandungsabschnitt kann in Richtung des Bodenrings 16 ein zweiter Wandungsabschnitt anschließen, dessen Innenwandung zylindrisch und koaxial zur Mittellängsachse L verläuft und dessen Außenwandung zum Bodenring 16 hin weiter nach außen gewölbt verläuft. An diesen, insbesondere mittleren, Wandungsabschnitt kann sich ein bodenseitiger Wandungsabschnitt anschließen. Vorzugsweise umfasst der bodenseitige Wandungsabschnitt den Bodenring 16. Bevorzugt verläuft in diesem Bereich die Innenwandung ausgehend von dem zylindrischen Mittelbereich zum Bodenabschnitt hin verjüngt zu, wodurch sich ein konisch nach unten verjüngt zulaufender Innenwandungsabschnitt ergibt.

Zur Stirnseite des Bodenrings 16 hin kann sich die Innenwandung wieder konisch unter Bildung einer Aufstandsfläche 18 erweitern. Über die Höhe des Ventilmantels 15 kann sich demzufolge eine eiförmige oder O-förmige Innenkontur 17 ergeben (siehe Fig. 4 und 5).

Eine in das Innere des Ventilmantels 15 weisende Innenfläche des Kopfbereichs 12 kann, wie in Fig. 5 dargestellt, als domartige Kontur 19 gestaltet sein.

Der Durchtritt 10 der Kappe 8 kann rotationssymmetrisch oder rotationsunsymmetrisch gestaltet sein. Bei einem rotationsunsymmetrischen Kopfbereich 12 ist vorzugsweise auch der Durchtritt 10 in komplementärer Weise rotationsunsymmetrisch gestaltet.

Wie anhand der Fig. 2 und 7 bis 9 erkennbar ist, ist der konischen Aufstandsfläche 18 des Bodenrings 16 des Ventilkörpers 11 im Bereich des Bodenabschnitts 7 eine komplementär konische Stützfläche 20 zugeordnet, wodurch sich der Ventilkörper 11 im Bereich des Bodenrings 16 über seine gesamte radiale Breite flächig auf dem Bodenabschnitt 7 abstützt.

Die Schlitzanordnung 14 ist quer zu einer Längserstreckung des Kopfbereichs 12 ausgerichtet, wie anhand der Fig. 4 bis 6 erkennbar ist. Die Schlitzanordnung 14 erstreckt sich vorzugsweise mittig längs der Mittellängsachse L durch den Kopfbereich 12 hindurch. Die Schlitzanordnung 14 weist zwei Schlitzwandungsflächen 21, 22 auf, die die Schlitzanordnung 14 in einem unbelasteten Ausgangszustand des Ventilkörpers 11 verschließen, vorzugsweise dicht.

Die Schlitzwandungsflächen 21, 22 der Schlitzanordnung 14 sind mit einer Zusammensetzung 23 versehen. Die Schlitzwandungsflächen 21, 22 können dabei nur teilweise oder vollständig, d.h. vollflächig, mit der Zusammensetzung 23 versehen, insbesondere beschichtet, sein.

Vorzugsweise befindet sich die Zusammensetzung23 nur auf den Schlitzwandungsflächen 21, 22 der Schlitzanordnung 14. Mit anderen Worten ist insbesondere der Kopfbereich 12 im Übrigen sowie der Ventilmantel 15 bevorzugt zusammensetzungsfrei, d.h. frei von der Zusammensetzung.

Durch die Zusammensetzung 23 lässt sich vorteilhafterweise eine strahleninduzierte Vernetzung des elastisch deformierbaren Materials des Ventilkörpers 11, beispielsweise während einer Gamma-Sterilisierung, Beta-Sterilisierung, Röntgen-Sterilisierung oder Elektronenstrahl-Sterilisierung (e-beam-Sterilisierung) der Verbindungseinrichtung 1, verhindern. Dadurch kann das Risiko eines Zuwachsens der Schlitzanordnung 14 signifikant reduziert oder sogar vollständig vermieden werden.

Die Zusammensetzung 23 weist ein Öl und einen Verdicker, vorzugsweise ein bioverträgliches Öl und einen bioverträglichen Verdicker, auf oder besteht aus einem Öl und einem Verdicker, vorzugsweise einem bioverträglichen Öl und einem bioverträglichen Verdicker. Bei dem Öl handelt es sich vorzugsweise um ein Silikonöl, insbesondere ein teil- oder perfluoriertes Silikonöl. Alternativ kann es sich bei dem Öl auch um ein fluorfreies Silikonöl handeln. Bei dem Verdicker handelt es sich vorzugsweise um Polytetrafluorethylen (PTFE) und/oder Silika. Vorzugsweise liegt die Zusammensetzung in Form einer zähviskosen oder pastösen Zusammensetzung vor. Besonders bevorzugt handelt es sich bei der Zusammensetzung um eine Schmierfettzusammensetzung.

Die vorgenannte Zusammensetzung hat sich im Hinblick auf die Vermeidung eines Zuwachsens bzw. Heilens der Schlitzanordnung 14 während einer Strahlensterilisierung, wie insbesondere Gammastrahlensterilisierung, als besonders geeignet herausgestellt. Ein weiterer Vorteil dieser Stoffe besteht insbesondere darin, dass sie eine etwaige Transparenz des Ventilkörpers 11 nicht beeinträchtigen. Obendrein können die vorgenannten Stoffe vorteilhafterweise zu einer Erhöhung der Alterungsresistenz des Ventilkörpers 11 beitragen.

Sobald eine Spitze einer mit dem Anschlussstutzen 3 zu konnektierenden Komponente K von außen her an den Anschlussstutzen 3 herangeführt wird, kommt die Spitze flächig mit der Außenfläche des Kopfbereichs 12 in Kontakt und drückt den Kopfbereich 12 in das Innere der Kappe 8 hinein. Dabei weitet sich die Schlitzanordnung 14, und elastisch deformierte Abschnitte des Kopfbereichs 12 legen sich bei einem weiteren Eindringen der Spitze der Komponente K außen an die Spitze an. Bei einem Dekonnektieren und einem daraus resultierenden Entfernen der Spitze nach außen stellt sich der Kopfbereich 12 wieder in die Ausgangslage gemäß Fig. 7 zurück.

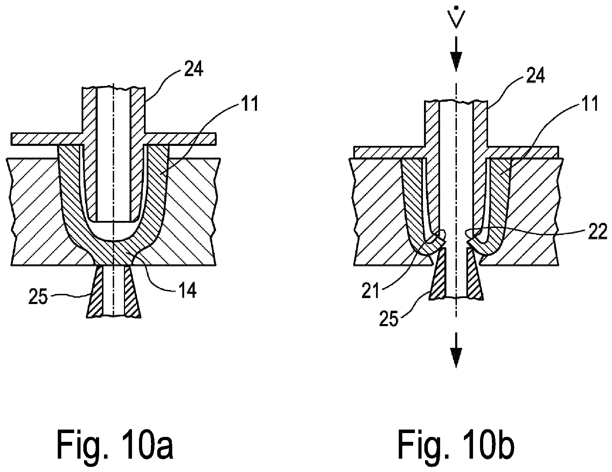

Die Figuren 10a und b zeigen schematisch eine Ausführungsform eines erfindungsgemäßen Verfahrens.

Bei dem Verfahren werden zunächst die Schlitzwandungsflächen 21, 22 der Schlitzanordnung 14 mit der Zusammensetzung beschichtet. Hierzu wird ein Ventilkörper 11 in eine Aufnahme oder unmittelbar auf eine Düse 24 platziert. Dabei kann die Düse 24 mit einem Niederhalter und/oder einer Kompressionsauflage angefahren werden, bis der Ventilkörper 11 komprimiert wird. Ferner kann der Ventilkörper 11, wie in den Fig. 10a und b dargestellt, gegen eine luerähnliche oder zylindrische Komponente 25 gedrückt werden, wobei ein wenigstens teilweises Öffnen des Ventilkörpers 11 bewirkt wird. Dabei kann die Komponente 25, wie in Fig. 10b dargestellt, etwas einfahren.

Bevorzugt weist die Düse 24 einen Durchmesser von 0.1 mm bis 10 mm, vorzugsweise 0.9 mm bis 2 mm, auf. Die vorgenannten Düsendurchmesser eignen sich besonders gut, um die Schlitzwandungsflächen 21, 22 der Schlitzanordnung 14 mit der Zusammensetzung zu beschichten und ein Zuwachsen der Schlitze durch die erfindungsgemäße Zusammensetzung zu verhindern. Ferner können die vorgenannten Düsendurchmesser eine Entmischung der Zusammensetzung, d.h. eine Trennung der Zusammensetzung in Öl und Verdicker, vorteilhafterweise verhindern.

Weiter kann die Düse 24 elliptisch oder als Schlitzdüse gestaltet sein. Ggf. kann eine Ausrichtung zu den Schlitzwandungsflächen 21, 22 der Schlitzanordnung 14 zweckmäßig sein.

Alternativ oder in Kombination können die Schlitzwandungsflächen 21, 22 der Schlitzanordnung 14 mit der Zusammensetzung mit Hilfe eines Streichelements, insbesondere einer Rakel, bestrichen oder eingestrichen werden.

Alternativ oder in Kombination kann eine Applikation der Zusammensetzung durch einen Luftstrom oder Nebel, insbesondere einen feuchten oder trockenen Nebel, erfolgen. Vorzugsweise wird der Luftstrom oder Nebel derart durch den teilweise oder vollständig geöffneten Ventilkörper 11 durchgeführt, dass der Luftstrom oder Nebel mit der Zusammensetzung die Schlitzwandungsflächen 21, 22 der Schlitzanordnung 14 beflutet. Die Beförderung der Zusammensetzung durch den Luftstrom oder Nebel erfolgt dabei in Pfeilrichtung (siehe Fig. 10b). Der Luft- oder Nebeldruck zum Befördern der Zusammensetzung zu den Schlitzwandungsflächen 21, 22 der Schlitzanordnung 14 kann von 0,1 bar bis 7 bar, vorzugsweise 0,5 bar bis 3 bar, gewählt werden. Ferner kann eine Volumenstromzeit von 1 ms bis 5 s, insbesondere 50 ms bis 1 s, gewählt werden.

Vorzugsweise wird der Ventilkörper 11 derart komprimiert und mithin geöffnet, dass das Innenlumen des Ventilkörpers 11 nichtbeschichtet wird.

## Patentansprüche

1. Medizinprodukt, aufweisend ein elastisch deformierbares Funktionsteil mit einer öffnungsfähigen Schlitzanordnung, wobei sich die Schlitzanordnung bei einer elastischen Deformation des elastisch deformierbaren Funktionsteils aufweitet oder öffnet und bei Wegfall der elastischen Deformation wieder schließt, **dadurch gekennzeichnet, dass** Schlitzwandungsflächen der Schlitzanordnung mit einer Zusammensetzung beschichtet sind, wobei die Zusammensetzung ein Silikonöl und einen Verdicker aufweist.

2. Medizinprodukt nach Anspruch 1, **dadurch gekennzeichnet, dass** das Silikonöl ein fluoriertes Silikonöl ist.

3. Medizinprodukt nach Anspruch 1 oder 2, **dadurch gekennzeichnet, dass** das Silikonöl einen Anteil von 0.1 Gew.-% bis 99.9 Gew.-%, insbesondere 10 Gew.-% bis 90 Gew.-%, vorzugsweise 20 Gew.-% bis 80 Gew.-%, aufweist, bezogen auf das Gesamtgewicht der Zusammensetzung.

4. Medizinprodukt nach einem der vorhergehenden Ansprüche, **dadurch gekennzeichnet, dass** der Verdicker ausgewählt ist aus der Gruppe bestehend aus Aluminiumseife, Aluminiumkomplexseife, Bariumseife, Bariumkomplexseife, Calciumseife, Calciumkomplexseife, Lithiumseife, Lithiumkomplexseife, Natriumseife, Natriumkomplexseife, Polytetrafluorethylen (PTFE), anorganischem Verdicker, insbesondere Bentonit, Polyharnstoff, Silika, Glucose, Ascorbinsäure, Stärke und Mischungen von wenigstens zwei der vorgenannten Verdicker.

5. Medizinprodukt nach einem der vorhergehenden Ansprüche, **dadurch gekennzeichnet, dass** der Verdicker Polytetrafluorethylen (PTFE), Silika oder eine Mischung aus Polytetrafluorethylen (PTFE) und Silika ist.

6. Medizinprodukt nach einem der vorhergehenden Ansprüche, **dadurch gekennzeichnet, dass** der Verdicker einen Anteil von 0.1 Gew.-% bis 99.9 Gew.%, insbesondere 10 Gew.-% bis 90 Gew.-%, vorzugsweise 20 Gew.-% bis 80 Gew.-%, aufweist, bezogen auf das Gesamtgewicht der Zusammensetzung.

7. Medizinprodukt nach einem der vorhergehenden Ansprüche, **dadurch gekennzeichnet, dass** die Zusammensetzung ferner einen Feststoff aufweist, der kein Verdicker ist, wobei der Feststoff insbesondere ausgewählt ist aus der Gruppe bestehend aus Salzen, Sacchariden, Vitaminen und Mischungen von wenigstens zwei der vorgenannten Feststoffe.

8. Medizinprodukt nach Anspruch 8, **dadurch gekennzeichnet, dass** der Feststoff einen Anteil von 0.01 Gew.-% bis 99.99 Gew.-%, insbesondere 1 Gew.-% bis 99 Gew.-%, vorzugsweise 10 Gew.-% bis 90 Gew.-%, aufweist, bezogen auf das Gesamtgewicht der Zusammensetzung.

9. Medizinprodukt nach einem der vorhergehenden Ansprüche, **dadurch gekennzeichnet, dass** die Zusammensetzung eine zähflüssige oder pastöse Zusammensetzung, vorzugsweise Schmierfettzusammensetzung, ist.

10. Medizinprodukt nach einem der vorhergehenden Ansprüche, **dadurch gekennzeichnet, dass** das elastisch deformierbare Funktionsteil wenigstens abschnittsweise transparent, d.h. lichtdurchlässig, gestaltet ist.

11. Medizinprodukt nach einem der vorhergehenden Ansprüche, **dadurch gekennzeichnet, dass** das elastisch deformierbare Funktionsteil aus einem elastisch deformierbaren Material, insbesondere Elastomer, vorzugsweise Silikon-Elastomer, und/oder thermoplastischen Elastomer, gefertigt ist.

12. Medizinprodukt nach einem der vorhergehenden Ansprüche, **dadurch gekennzeichnet, dass** die Schlitzanordnung als Längsschlitzanordnung gestaltet ist.

13. Medizinprodukt nach einem der vorhergehenden Ansprüche, **dadurch gekennzeichnet, dass** das elastisch deformierbare Funktionsteil als Ventilkörper gestaltet ist.

14. Medizinprodukt nach einem der vorhergehenden Ansprüche, **dadurch gekennzeichnet, dass** das elastisch deformierbare Funktionsteil einen Kopfbereich aufweist, wobei die Schlitzanordnung entlang einer Quererstreckung des Kopfbereiches ausgebildet ist und sich mittig längs einer Mittellängsachse durch den Kopfbereich hindurch erstreckt.

15. Medizinprodukt nach einem der vorhergehenden Ansprüche, **dadurch gekennzeichnet, dass** es sich bei dem Medizinprodukt um ein Medizinprodukt für die Infusionstherapie, insbesondere um eine Verbindungseinrichtung, insbesondere in Form eines Einwege-, Zweiwege-, Dreiwege- oder Vierwegehahns oder einer Hahnbank, eines medizinischen Infusionssystems, handelt.

16. Elastisch deformierbares Funktionsteil für ein Medizinprodukt, **dadurch gekennzeichnet, dass** das elastisch deformierbare Funktionsteil nach wenigstens einem der vorhergehenden Ansprüche gestaltet ist.

17. Verfahren zum Sterilisieren und/oder zum Herstellen von Sterilisationsbeständigkeit eines Medizinprodukts nach einem der Ansprüche 1 bis 16 oder eines elastisch deformierbaren Funktionsteils nach Anspruch 17, **dadurch gekennzeichnet, dass** das Verfahren, vorzugsweise in zeitlicher Reihenfolge, nachfolgende Schritte aufweist:
a) Beschichten oder Beaufschlagen der Schlitzwandungsflächen der Schlitzanordnung mit der Zusammensetzung, aufweisend ein Silikonöl und einen Verdicker, und
b) Sterilisieren des Medizinprodukts oder elastisch deformierbaren Funktionsteils, vorzugsweise durch Einwirkung von Gamma-, Röntgen-, Beta- oder Elektronenstrahl-Strahlung.
